# EUROPÄISCHE PATENTSCHRIFT

(11) **EP 2 262 546 B1**
(45) Veröffentlichungstag und Bekanntmachung des Hinweises auf die Patenterteilung: **24.10.2012**
(21) Anmeldenummer: 09718362.8
(22) Anmeldetag: 21.02.2009
(51) Int. Cl.: A61L 24/04, C08G 18/10, C09J 175/02, C09J 175/04, A61L 24/00

(54) **MEDIZINISCHE KLEBSTOFFE FÜR DIE CHIRURGIE MIT BIOAKTIVEN VERBINDUNGEN**
MEDICAL GLUES FOR SURGERY COMPRISING BIOACTIVE COMPOUNDS
ADHÉSIF MÉDICAL POUR LA CHIRURGIE AVEC DES COMPOSÉS BIOACTIFS

(30) Priorität: 06.03.2008 EP 08004134
(43) Veröffentlichungstag der Anmeldung: 22.12.2010
(73) Patentinhaber: Bayer MaterialScience AG, 51368 Leverkusen (DE)
(72) Erfinder: HECKROTH, Heike, 51519 Odenthal (DE); KÖHLER, Burkhard, 34289 Zierenberg (DE); DÖRR, Sebastian, 40597 Düsseldorf (DE)
(86) Internationale Anmeldenummer: PCT/EP2009/001262
(87) Internationale Veröffentlichungsnummer: WO 2009/109306

(56) Entgegenhaltungen:
- EP-A- 0 488 629
- EP-A- 1 081 171
- EP-A- 1 719 530
- WO-A-2007/067761
- US-A- 4 477 604
- US-A1- 2003 232 954
- US-A1- 2004 067 315
- US-A1- 2004 115 229

## Beschreibung

Die vorliegende Erfindung betrifft neuartige, schnell härtende Klebstoffe auf Basis hydrophiler Polyisocyanat-Prepolymere für den Einsatz in der Chirurgie, die pharmakologisch aktive Wirkstoffe enthalten.

In den vergangenen Jahren hat sich ein steigendes Interesse entwickelt, chirurgische Nähte durch den Einsatz von geeigneten Klebstoffen zu ersetzen oder zu unterstützen. Besonders im Bereich der plastischen Chirurgie, in der auf dünne, möglichst unsichtbare Narben Wert gelegt wird, werden vermehrt Klebstoffe eingesetzt.

Gewebekleber müssen eine Reihe von Eigenschaften haben, um bei den Chirurgen als Nahtersatz akzeptiert zu werden. Hierzu gehören eine leichte Verarbeitbarkeit und eine Eingangsviskosität, so dass der Klebstoff nicht in tiefere Gewebeschichten eindringen oder verlaufen kann. In der klassischen Chirurgie wird eine schnelle Aushärtung erfordert, wogegen in der plastischen Chirurgie eine Korrektur der Klebenaht möglich sein sollte und damit die Aushärtungsgeschwindigkeit nicht zu schnell sein darf (ca. 5 min). Die Klebeschicht sollte ein flexibler, transparenter Film sein, der nicht in einem Zeitraum kleiner drei Wochen abgebaut wird. Der Klebstoff muß biokompatibel sein und darf weder Histotoxicität noch Thrombogenität oder ein allergenes Potential besitzen.

Diverse Materialien, die als Gewebekleber eingesetzt werden, sind im Handel erhältlich. Hierzu gehören die Cyanacrylate Dermabond® (Octyl-2-Cyanoacrylat) und Histoacryl Blue® (Butyl-Cyanoacrylat). Die schnelle Aushärtezeit, sowie die Sprödigkeit der Klebestelle limitieren jedoch den Einsatz. Durch die schlechte Bioabbaubarkeit sind Cyanacrylate nur für äußerliche chirurgische Nähte geeignet.

Als Alternative zu den Cyanacrylaten stehen biologische Klebstoffe wie peptidbasierte Substanzen (BioGlue^{®}) oder Fibrinkleber (Tissucol) zur Verfügung. Neben den hohen Kosten zeichnen sich Fibrinkleber durch eine relative schwache Klebstärke und einen schnellen Abbau aus, so dass dieser nur bei kleineren Schnitten auf nicht gespannter Haut anwendbar ist.

Die in US 20030135238 und US 20050129733 beschriebenen Isocyanat-haltigen Klebstoffe basieren auf einem aromatischen Diisocyanat und einem hydrophilen Polyol, wobei bevorzugt die Isocyanate TDI und MDI eingesetzt werden. Beide können, um die Reaktivität zu erhöhen, Elektronen ziehende Substituenten tragen (WO-A 03/9323).

Die Ausrüstung des darin beschriebenen Klebstoffes mit Wirkstoffen ist nun für verschiedene Gebiete interessant. Durch Verwendung von Analgetika wird das Schmerzempfinden an der zu behandelnden Stelle herabgesetzt oder ausgeschaltet, wodurch auf eine subcutane Injektion eines Analgetikums verzichtet werden kann. Besonders im Bereich der Tiermedizin, in der bei topischen Schnitten wie Kastrationen oder dem Mulesing bei Schafen nur selten Schmerzmittel eingesetzt werden, ist ein im Klebstoff integriertes Analgetikum indiziert. Durch Herabsetzung des Schmerzempfindens wird zudem die Gefahr eines traumatischen Schocks reduziert.

Die Verwendung von antimikrobiell/antiseptisch wirksamen Substanzen verhindert ein Eindringen von Keimen in die Wunde bzw. bewirkt ein Abtöten bereits vorhandener Bakterien. Dies ist speziell in der Tiermedizin von Interesse, da hier nur in seltenen Fällen aseptisch gearbeitet werden kann. Das gleiche gilt für antimykotisch wirkende Verbindungen.

Das Aufbringen bioaktiver Verbindungen auf die intakte Haut ist in Form von selbstklebenden Wirkstoffpflastern dem Fachmann bekannt und unter anderem in WO 2005/046654, WO 2005/046653 und WO 2004/110428 beschrieben. Die aktive Verbindung ist hier jedoch nicht im Klebstoff integriert. WO 2006/102385 und EP-A 1719530 erwähnen allgemein die Verwendung bioaktiver Agentien bei der Anwendung von Cyanacrylaten und Polyurethanen als chirurgische Klebstoffe.

Die Patente US 5,684,042, US 5,753,699, US 5,762,919, US 5,783177, US 5,811091, US 6,902594 und EP-A 1508601 beschreiben Cyanacrylate, bei denen als antimikrobiell wirksame Substanz Iod bzw. Iodkomplexe wie Polyvinylpyrrolidon-Iod eingesetzt werden.

US 2003/0007947 beschreibt die Verwendung von Antimykotika in Cyanacrylat-Klebstoffen zur Behandlung oraler Candidasen, US 2003/0007948 bezieht sich auf cutane Candidasen.

Es wurde nun gefunden, dass sich die in den nicht vorveröffentlichten Europäischen Patentanmeldungen Nr. 07021764.1 und Nr. 08001290.9 beschriebenen Wundkleber auf Basis einer Kombination hydrophiler aliphatischer Polyisocyanat-Prepolymere und Aspartaten als Härter ebenfalls mit Wirkstoffen ausrüsten lassen und die so resultierenden Klebstofffilme eine Freisetzung der Wirkstoffe zulassen.

Gegenstand der vorliegenden Erfindung sind daher Klebstoffsysteme umfassend
A) isocyanatfunktionelle Prepolymere erhältlich aus
   A1) aliphatischen Isocyanaten und
   A2) Polyolen mit zahlenmittleren Molekulargewichten von ≥400 g/mol und mittleren OH-Funktionalitäten von 2 bis 6
B) eine Härterkomponente umfassend
   B1)aminofunktionelle Asparaginsäureester der allgemeinen Formel (I) 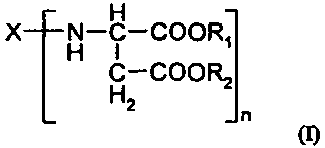 wobei
      - X: ein n-wertiger organischer Rest ist, der durch Entfernung der primären Aminogruppen eines n-wertigen Amins erhalten wird,
      - R₁, R₂: gleiche oder verschiedene organische Reste sind, die keinen Zerewitinoff-aktiven Wasserstoff aufweisen und
      - n: eine ganze Zahl von mindestens 2 ist
      und B2)organische Füllstoffe, die eine nach DIN 53019 gemessenen Viskosität bei 23°C im Bereich von 10 bis 6000 mPas aufweisen
C) gegebenenfalls Umsetzungsprodukte von isocyanatfunktionellen Prepolymeren gemäß der Definition der Komponente A) mit Asparaginsäureestern gemäß Komponente B1) und/oder organischen Füllstoffen gemäß Komponente B2)
   und
D) mindestens eine pharmakologisch aktive Verbindung, dadurch gekennzeichnet, dass es sich um einen Gewebekleber für menschliches oder tierisches Gewebe handelt.

Zur Definition von Zerewitinoff-aktivem Wasserstoff wird auf Römpp Chemie Lexikon, Georg Thieme Verlag Stuttgart verwiesen. Bevorzugt werden unter Gruppen mit Zerewitinoff-aktivem Wasserstoff OH, NH oder SH verstanden.

Unter Gewebe im Rahmen der vorliegenden Erfindung werden Zellverbände verstanden, die aus Zellen gleicher Gestalt und Leistung bestehen wie Deckgewebe (Haut), Epithelgewebe, Myocard, Binde- oder Stützgewebe, Muskeln, Nerven und Knorpel. Hierzu gehören unter anderem auch alle aus Zellverbänden aufgebaute Organe wie Leber, Niere, Lunge, Herz etc.

Unter pharmakologisch aktiven Verbindungen versteht man allgemein Stoffe und Zubereitungen aus Stoffen, die zur Anwendung am oder im menschlichen oder tierischen Körper bestimmt sind, um Krankheiten, Leiden, Körperschäden oder krankhafte Beschwerden zu heilen, zu lindern, zu verhüten oder zu erkennen. Darunter fallen ebenfalls Stoffe und Zubereitungen daraus, um Krankheitserreger, Parasiten oder körperfremde Stoffe abzuwehren, zu beseitigen oder unschädlich zu machen.

Analgetika sind schmerzstillende Substanzen unterschiedlicher chemischer Struktur und Wirkungsweise.

Antiphlogistika sind entzündungshemmende Substanzen.

Antimikrobiell wirksame Substanzen/Antiseptika sind Verbindungen, die das Wachstum bestimmter Mikroorganismen wie z. B. Bakterien, Pilze, Algen und Protozoen inhibiert und/oder diese absterben lässt.

Antimykotika sind Arzneimittel zur Behandlung von Pilzinfektionen.

Antiparasitär wirksame Verbindungen sind Wirkstoffe, die Parasiten abtöten oder deren Wachstum sowie das Einnisten in menschlichem oder tierischen Gewebe inhibieren bzw. verhindern.

Die in A) eingesetzten isocyanatfunktionellen Prepolymere sind durch Umsetzung von Isocyanaten mit hydroxyfunktionellen Polyolen gegebenenfalls unter Zusatz von Katalysatoren sowie Hilfs-und Zusatzstoffen erhältlich.

In A1) können als Isocyanate beispielsweise monomere aliphatische oder cycloaliphatische Di-oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonan-triisocyanat), sowie Alkyl-2,6-diisocyanatohexanoate (Lysindiisocyanat) mit C1-C8-Alkylgruppen eingesetzt werden.

Neben den vorstehend genannten monomeren Isocyanaten können auch deren höhermolekulare Folgeprodukte mit Uretdion-, Isocyanurat-, Urethan-, Allophanat-, Biuret-, Iminooxadiazindion-oder Oxadiazintrionstruktur sowie deren Mischungen eingesetzt werden.

Bevorzugt werden in A1) Isocyanate der vorstehend genannten Art mit ausschließlich aliphatisch oder cycloaliphatisch gebundenen Isocyanatgruppen oder deren Mischungen eingesetzt.

Die in A1) eingesetzten Isocyanate oder Isocyanatmischungen haben bevorzugt eine mittlere NCO-Funktionalität von 2 bis 4, besonders bevorzugt 2 bis 2,6 und ganz besonders bevorzugt 2 bis 2,4.

In einer besonders bevorzugten Ausführungsform wird in A1) Hexamethylendiisocyanat eingesetzt.

Zum Prepolymeraufbau können in A2) grundsätzliche alle dem Fachmann an sich bekannten Polyhydroxyverbindungen mit 2 oder mehr OH-Funktionen pro Molekül eingesetzt werden. Dies können beispielsweise Polyesterpolyole, Polyacrylatpolyole, Polyurethanpolyole, Polycarbonatpolyole, Polyetherpolyole, Polyesterpolacrylatpolyole, Polyurethanpolyacryl-atpolyole, Polyurethanpolyesterpolyole, Polyurethanpolyetherpolyole, Polyurethanpolycarbonat-polyole, Polyesterpolycarbonatpolyole oder deren beliebige Mischungen untereinander sein.

Die in A2) eingesetzten Polyole haben bevorzugt eine mittlere OH-Funktionalität von 3 bis 4

Die in A2) eingesetzten Polyole haben ferner bevorzugt ein zahlenmittleres Molekulargewicht von 400 bis 20000 g/mol, besonders bevorzugt 2000 bis 10000 g/mol und ganz besonders bevorzugt 4000 bis 8500.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Bevorzugte Polyalkylenoxid-Polyether entsprechen denen der vorstehend genannten Art und weisen Gehalt an Ethylenoxid-basierten Einheiten von 50 bis 100 %, bevorzugt 60 bis 90 % bezogen auf die insgesamt enthaltene Mengen an Alkylenoxideinheiten auf.

Bevorzugte Polyesterpolyol sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbemsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können hydroxylgruppenaufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Bevorzugt werden zum Prepolymeraufbau Polyetherpolyole der vorstehend genannten Art eingesetzt.

Zur Herstellung des Prepolymers werden die Verbindungen der Komponente A1) mit denen der Komponente A2) bei einem NCO/OH-Verhältnis von bevorzugt 4:1 bis 12:1, besonders bevorzugt 8:1 umgesetzt und anschließend der Anteil an nicht umgesetzten Verbindungen der Komponente A1) mittels geeigneter Methoden abgetrennt. Üblicherweise wird hierfür die Dünnschichtdestillation verwendet, wobei restmonomerenarme Produkte mit Restmonomergehalten von weniger als 1 Gew.-%, bevorzugt weniger als 0,5 Gew.-%, ganz besonders bevorzugt weniger als 0,1 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur beträgt dabei 20 bis 120°C, bevorzugt 60 bis 100°C.

Bevorzugt sind in Formel (I):
- R₁, R₂: gleiche oder verschiedene gegebenenfalls verzweigte oder cyclische organische Reste mit 1 bis 20, bevorzugt 1 bis 10 Kohlenstoffatomen, die keinen Zerewitinoff-aktiven Wasserstoff aufweisen,
- n: eine ganze Zahl von 2 bis 4 und
- X: ein n-wertiger organischer gegebenenfalls verzweigter oder cyclischer organischer Rest mit 2 bis 20, bevorzugt 5 bis 10 Kohlenstoffatomen, der durch Entfernung der primären Aminogruppen eines n-wertigen primären Amins erhalten wird.

Die Herstellung der aminofunktionellen Polyasparaginsäweester B1) erfolgt in bekannter Weise durch Umsetzung der entsprechenden primären mindestens difunktionellen Amine X(NH₂)ₙ mit Malein- oder Fumarsäureestern der allgemeinen Formel

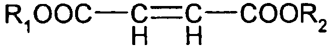

Bevorzugte Malein- oder Fumarsäureester sind Maleinsäuredimethylester, Maleinsäurediethylester, Maleinsäuredibutylester und die entsprechenden Fumarsäureester.

Bevorzugte primäre mindestens difunktionelle Amine X(NH₂)ₙ sind Ethylendiamin, 1,2-Diaminopropan, 1,4-Diaminobutan, 1,3-Diaminopentan, 1,5-Diaminopentan, 2-Methyl-1,5-Diaminopentan, 1,6-Diaminohexan, 2;5-Diamino-2,5-dimethylhexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethyl-cyclohexan, 2,4-und/oder 2,6-Hexahydrotoluylendiamin, 2,4'-und/oder 4,4'-Diamino-dicyclohexylmethan, 3,3'-Dimethyl-4,4'-diamino-dicyclohexyl-methan, 2,4,4'-Triamino-5-methyl-dicyclohexylmethan und Polyetheramine mit aliphatisch gebundenen primären Aminogruppen mit einem zahlenmittleren Molekulargewicht Mₙ von 148 bis 6000 g/mol.

Besonders bevorzugte primäre mindestens difunktionelle Amine sind 1,3-Diaminopentan, 1,5-Diaminopentan, 2-Methyl-1,5-diaminopentan, 1,6-Diaminohexan, 1,13-Diamino-4,7,10-trioxatridecan. Ganz besonders bevorzugt ist 2-Methyl-1,5-diaminopentan.

Bevorzugt sind R₁ und R₂ unabhängig voneinander C₁ bis C₁₀-Alkylreste, besonders bevorzugt Methyl oder Ethylreste.

In einer bevorzugten Ausführungsform der Erfindung sind R₁ = R₂ = Ethyl, wobei X auf 2-Methyl-1,5-Diaminopentan, als n-wertigem Amin basiert.

Bevorzugt ist n in Formel (I) für die Beschreibung der Wertigkeit des n-wertigen Amins eine ganze Zahl von 2 bis 6, besonders bevorzugt 2 bis 4.

Die Herstellung der aminofunktionellen Asparaginsäureester B1) aus den genannten Ausgangsmaterialien erfolgt nach DE-A 69 311 633 bevorzugt innerhalb des Temperaturbereichs von 0 bis 100 °C, wobei die Ausgangsmaterialien in solchen Mengenverhältnissen eingesetzt werden, dass auf jede primäre Aminogruppe mindestens eine, vorzugsweise genau eine olefinische Doppelbindung entfällt, wobei im Anschluss an die Umsetzung gegebenenfalls im Überschuss eingesetzte Ausgangsmaterialien destillativ abgetrennt werden können. Die Umsetzung kann in Substanz oder in Gegenwart geeigneter Lösungsmittel wie Methanol, Ethanol, Propanol oder Dioxan oder Gemischen derartiger Lösungsmittel erfolgen.

Die in B2) eingesetzten organischen flüssigen Füllstoffe sind bevorzugt gemäß Zytotoxizitätsmessung nach ISO 10993 nicht zytotoxisch.

Beispielsweise können als organische Füllstoffe flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Bevorzugt handelt es sich bei den organischen Füllstoffen der Komponente B2) um hydroxy- oder aminofunktionelle, bevorzugt rein hydroxyfunktionelle Verbindungen. Bevorzugte rein hydroxyfunktionelle Verbindungen sind Polyether und/oder Polyesterpolyole, besonders bevorzugt Polyetherpolyole.

Die bevorzugten organischen Füllstoffe der Komponente B2) besitzen bevorzugt mittlere OH-Funktionalitäten von 1,5 bis 3, besonders bevorzugt 1,8 bis 2,2, ganz besonders bevorzugt 2,0.

Die bevorzugten organischen Füllstoffe der Komponente B2) besitzen bevorzugt sich wiederholende von Ethylenoxid abgeleitete Einheiten.

Die Viskosität der organischen Füllstoffe der Komponente B2) beträgt bevorzugt 50 bis 4000 mPas bei 23°C gemessen nach DIN 53019.

In einer bevorzugten Ausführungsform der Erfindung werden als organische Füllstoffe der Komponente B2) Polyethylenglycole eingesetzt. Diese haben bevorzugt ein zahlenmittleres Molekulargewicht von 100 bis 1000 g/mol, besonders bevorzugt 200 bis 400 g/mol.

Das Gewichtsverhältnis von B1) zu B2) beträgt 1:0 bis 1:20 , bevorzugt 1:0 bis 1:12.

Das Gewichtsverhältnis der Komponente B2 bezogen auf die Gesamtmenge der Mischung aus B1, B2 und A liegt im Bereich von 0 bis 100 %, bevorzugt 0 bis 60 %.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich zu den in B1) und B2) eingesetzten Verbindungen auch die amino- oder hydroxyfunktionellen Umsetzungsprodukte isocyanatfunktioneller Prepolymere mit Asparaginsäureestern und/oder organischen Füllstoffen B2), sofern diese amino- oder hydroxyfunktionell sind, in einer separaten Vorreaktion herzustellen und dann als höhermolekulare Härterkomponente C) einzusetzen.

Bevorzugt werden bei der Vorverlängerung Verhältnisse von isocyanatreaktiven Gruppen zu Isocyanatgruppen von 50 zu 1 bis 1,5 zu 1 eingesetzt, besonders bevorzugt 15 zu 1 bis 4 zu 1.

Das dafür einzusetzende isocyanatfunktionelle Prepolymer kann dabei demjenigen der Komponente A) entsprechen oder aber auch abweichend aus den Komponenten, wie sie als mögliche Bestandteile der isocyanatfunktionellen Prepolymere im Rahmen dieser Anmeldung gelistet sind, aufgebaut sein.

Vorteil dieses Modifizierung durch Vorverlängerung ist, dass das Equivalentgewicht und Equivalentvolumen der Härterkomponente in deutlichen Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden.

Pharmakologisch aktive Wirkstoffe können unter anderem aber nicht ausschließlich sein:
a) Analgetika mit und ohne antiinflammatorische Wirkung
b) Antiphlogistika
c) Antimikrobiell wirksame Substanzen
d) Antimykotika
e) Antiparasitär wirkende Stoffe

Der Wirkstoff ist bevorzugt bei Raumtemperatur in der Härterkomponente B löslich, kann aber auch in B suspendiert eingesetzt werden. In einer bevorzugten Ausführungsform der Erfindung wird der Wirkstoff in einer Mischung aus Härter B1 und Füllstoff B2 gelöst oder suspendiert, wobei als B2 bevorzugt Polyethylenglycole mit einem zahlenmittleren Molekulargewicht von 100 bis 1000 g/mol, besonders bevorzugt von 200 bis 400 g/mol eingesetzt werden.

Die Konzentration des zugesetzten Wirkstoffes richtet sich nach den therapeutisch notwendigen Dosen und liegt bei 0,001 Gew.-% bis 10 Gew.-%, bevorzugt bei 0,01 Gew.-% bis 5 Gew.-% bezogen auf die Gesamtmenge aller nichtflüchtigen Komponenten des Klebstoffsystems

Alle einsetzbaren Wirkstoffe zeichnen sich dadurch aus, dass sie nicht über NCO-reaktive funktionelle Gruppen verfügen, bzw. dass die Reaktion eventuell vorhandener funktioneller Gruppen mit dem Isocyanat-Prepolymer gegenüber der Aspartat- NCO-Reaktion deutlich langsamer ist.

Analgetika, die diesen Anspruch erfüllen, sind Lokalanästhetika wie Ambucain, Amylocain, Arecaidin, Benoxinat, Benzocain, Betoxycain, Butacain, Butethamin, Bupivacain, Butoxycain, Chlorprocain, Cocaethylen, Cocaine, Cyclomethycain, Dibucain, Dimethocain, Dimethisoquin, Etidocain, Fomacain, Isobutyl-p-aminobenzoat, Leucinocain, Lidocain, Meperidin, Mepivacain, Metabutoxycain, Octacain, Orthocain, Oxethazain, Phenacain, Piperocain, Piridocain, Pramoxin, Procain, Procainamid, Proparacain, Propoxycain, Pseudococain, Pyrrocain, Ropivacain, Tetracain, Tolycain, Tricain, Trimecain, Tropacocain, Amolanon, Cinnamoylcocain, Paretoxycain, Propiocain, Myrtecain und Propanocain.

Ebenso eingesetzt werden können Opioid-Analgetika wie Morphin und seine Derivate (z. B. Codein, Diamorphin, Dihydrocodein, Hydromorphon, Oxycodon, Hydrocodon, Buprenorphin, Nalbuphin, Pentazocin), Pethidin, Levomethadon, Tilidin und Tramadol.

Gleichfalls können nichtsteroidale Antiphlogistika (NSAID) wie Acetylsalicylsäure, Acemetacin, Dexketoprofen, Diclofenac, Aceclophenac, Diflunisal, Piritramid, Etofenamat, Felbinac, Flurbiprofen, Flufeaminsäure, Ibuprofen, Indometacin, Ketoprofen, Lonazolac, Lornoxicam, Mefenaminsäure, Meloxicam, Naproxen, Piroxicam, Tiaprofensäure, Tenoxicam, Phenylbutazon, Propyphenazon, Phenazon und Etoricoxib verwendet werden. Andere Analgetika wie Azapropazon, Metamizol, Nabumeton, Nefopam, Oxacephrol, Paracetamol sowie das analgetisch wirksame Amitriptylin können selbstverständlich ebenfalls eingesetzt werden.

Neben den genannten Analgetika, die eine entzündungshemmende Wirkung haben, können zusätzlich rein antiphlogistisch wirksame Verbindungen eingesetzt werden. Dazu gehört die Klasse der Glucocorticoide wie z. B. Cortison, Betamethason, Dexamethason, Hydrocortison, Methylprednisolon, Prednisolon, Prednison, Budesonid, Allotetrahydro-cortison, Fludrocortison, Fluprednisolon, Fluticasonpropionat, etc.

Als antiseptisch wirksame Substanzen können unter anderem die folgenden Verbindungen eingesetzt werden: Triclosan (2,4,4'-Trichloro-2'hydroxydiphenyl ether), Chlorhexidin und seine Salze, Octenidin, Chloramphenicol, Florfenicol, Chlorchinaldol, Iod, Povidon-Iod, Hexachlorophen, Merbromin, PHMB, nanokristallin vorliegendes Silber sowie Silber- und Kupfersalze.

Des weiteren können als antimikrobiell wirksame Substanzen Antibiotika aus der Klasse der β-Lactame (z. B. Penicillin und seine Derivate, Cephalosporine), der Tetracycline (z. B. Demeclocyclin, Doxycyclin, Oxytetracycline, Minocyclin, Tetracyclin), der Makrolide (z. B. Erythromycin, Josamycin, Spiramycin), der Lincosamide (z. B. Clindamycin, Lincomycin), der Oxazolidinone (z. B. Linezolid), der Gyrasehemmer (z. B. Danofloxacin, Difloxacin, Enrofloxacin, Ibafloxacin, Marbofloxacin, Nalidixinsäure, Pefloxacin, Fleroxacin, Levofloxacin) und der zyklischen Peptide (z. B. Bicozamycin) verwendet werden. Ebenso eingesetzt werden können Rifamycin, Rifaximin, Methenamin; Mupirocin, Fusidinsäure, Flumechin, und die Derivate des Nitroimidazols (z. B. Metronidazol, Nimorazol, Tinidazol), des Nitrofurans (Furaltadon, Nifurpirinol, Nihydrazone, Nitrofurantoin), des Sulfonamids (z. B. Sulfabromomethazin, Sulfacetamid, Sulfachlorpyridazine, Sulfadiazine etc.) sowie β-Lactamase-Inhibitoren wie die Clavulansäure.

Als antimykotisch wirksame Substanzen können alle Azolderivate, die die Biosynthese von Ergosterol hemmen, wie z. B. Clotrimazol, Fluconazol, Miconazol, Bifonazol, Econazol, Fenticonazol, Isoconazol, Oxiconazol etc. eingesetzt werden. Sonstige lokal anwendbare Antimykotika sind Amorolfin, Ciclopirox, Thymol und seine Derivate sowie Naftifin. Ebenso kann die Klasse der Alkylparabene eingesetzt werden.

Zu den antiparasitär wirksamen Verbindungen gehören unter anderem die Ektoparasitizide Cyfluthrin und Lindan, verschiedene Azolderivate wie z. B. Dimetridazol und Metronidazol sowie Chinin.

Bei Bedarf kann die Härterkomponente angefärbt werden.

Die erfindungsgemäßen 2-komponentigen Klebstoffsysteme werden durch Mischung des Prepolymers mit der Härterkomponente B) bzw. C) erhalten. In Komponente A), B) bzw. C) befindet sich die biologisch aktive Komponente D), bevorzugt in B) bzw. C). Das Verhältnis von NCO-reaktiven NH-Gruppen zu freien NCO-Gruppen beträgt bevorzugt 1:1,5 bis 1:1, besonders bevorzugt 1:1.

Die erfindungsgemäßen 2-komponentigen Klebstoffsysteme besitzen unmittelbar nach Vermischung der Einzelkomponenten miteinander eine Scherviskosität bei 23°C von bevorzugt 1000 bis 10000 mPas, besonders bevorzugt 2000 bis 8000 mPas und ganz besonders bevorzugt 2500 bis 5000 mPas.

Die Geschwindigkeit bei 23°C bis eine vollständige Vernetzung und Aushärtung des Klebestoffs erreicht ist, beträgt typischerweise 30 s bis 10 min, bevorzugt 1 min bis 8 min.

Ein weiterer Gegenstand der Erfindung sind die aus den erfindungsgemäßen Klebstoffsystemen erhältlichen Klebfilme sowie daraus hergestellte Verbundteile.

In einer bevorzugten Ausführungsform werden die erfindungsgemäßen 2-komponentigen Klebstoffsysteme als Gewebekleber für den Verschluss von Wunden in menschlichen oder tierischen Zellverbänden verwendet, so dass auf ein Klammem oder Nähen zum Verschließen weitestgehend verzichtet werden kann.

Der erfindungsgemäße Gewebekleber kann so wohl in vivo als auch in vitro angewendet werden, wobei die in vivo Anwendung beispielsweise zur Wundbehandlung nach Unfällen oder Operationen bevorzugt ist.

Ebenfalls Gegenstand der Erfindung ist ferner die Verwendung solcher 2-komponentigen Klebstoffsysteme zur Herstellung eines Mittels zum Verschließen oder Verbinden von Zellgeweben, so wie die zur Applikation notwendigen 2-Kammerdosiersysteme umfassend die erfindungswesentlichen Komponenten des Klebstoffsystems.

### Beispiele:

Sofern nicht abweichend angegeben beziehen sich alle Prozentangaben auf das Gewicht.

Zur Verklebung *in vitro* wurde als Gewebe Rind- oder Schweinefleisch verwendet. Es wurden jeweils zwei Stücke Fleisch (1 = 4 cm, h = 0.3 cm, b = 1 cm) an den Enden 1 cm weit mit dem Kleber bestrichen und überlappend geklebt. Die Stabilität der Klebeschicht wurde jeweils durch Zug überprüft.
PEG = Polyethylenglykol

### Beispiel 1, (Prepolymer A)

465 g HDI und 2.35 g Benzoylchlorid wurden in einem 1 1 Vierhalskolben vorgelegt. Innerhalb von 2h wurden bei 80°C 931.8 g eines Polyethers mit einem Ethylenoxidgehalt von 63% und einem Propylenoxidgehalt von 37% (jeweils bezogen auf dem gesamten Alkylenoxidgehalt) gestartet auf TMP (3-funktionell) hinzugefügt und rührte 1h nach. Anschließend wurde durch Dünnschichtdestillation bei 130°C und 0,1 Torr das überschüssige HDI abdestilliert. Man erhielt 980 g (71 %) des Präpolymers mit einem NCO-Gehalt von 2,53%. Der Restmonomerengehalt betrug < 0,03 % HDI.

### Beispiel 2, (Aspartat B)

Zu 2 Mol Diethylmaleat wurde unter Stickstoffatmosphäre langsam 1 Mol 2-Methyl-1,5-diaminopentan getropft, so dass die Reaktionstemperatur 60°C nicht überschritt. Anschließend wurde so lange auf 60°C erwärmt, bis kein Diethylmaleat mehr im Reaktionsgemisch nachweisbar war. Das Produkt wurde durch Destillation gereinigt.

### Beispiele zur Klebung von Gewebe mit Wirkstoffen:

### Beispiel 3, in vitro Klebung von Muskelgewebe

0,45 g PEG 200 wurden mit 0,55 g Aspartat B und 2-5 % des Wirkstoffs gut vermischt. Die Lösung wurde mit 4 g Prepolymer A verrührt und auf das Gewebe aufgetragen. Verarbeitungszeit und Klebewirkung auf Fleisch sowie die Filmbildung auf Haut wurden untersucht.

**Tabelle 1**

| **Wirkstoff** | **Topfzeit** | **Klebestärke¹ [nach 4 min]** | **Aushärtungszeit Filmbildung [min bis zum Verschwinden der Klebrigkeit der Oberfläche]** |
|---|---|---|---|
| ohne | 1 min 10 s | ++ | 3 |
| Lidocain | 1 min 30 s | ++ | 4 |
| Acemetacin | 1 min 30 s | + | 7 |
| Benzocain | 1 min 30 s | + | 6 |
| Tetracain | 1 min 40 s | + | 7 |
| Phenylbutazon | 1 min 30 s | + | 8 |
| Paracetamol | 1 min 30 s | + | 7 |
| Ibuprophen | 1 min 30 s | ++ | 6 |
| Erythromycin | 1 min 30 s | ++ | 6 |
| Nalidixinsäure² | 1 min 30 s | ++ | 5 |
| Chlorhexidin | 30 s | ++ | 3 |
| Triclosan (Irgasan) | 1 min 30 s | ++ | 5 |
| Thymol | 1 min 30 s | ++ | 5 |
| Fluconazol | 3 min | [3] | 15 |
| Metronidazol² | 2 min | ++ | 5 |
| Cortison² | 1 min 20 s | ++ | 5 |
| Furaldaton² | 1 min 30 s | + | 4 |
| Sulfacetamid | 1 min 15 s | ++ | 5 |
| Enrofloxazin² | 1 min | ++ | 4 |
| Chloramphenicol | 1 min 30 s | ++ | 5 |
| Tetracyclin | 1 min 15 s | ++ | 4 |
| Acetylsalicylsäure | 1 min 30 s | ++ | 4 |
| Amitriptylin | 1 min 20 s | ++ | 3 min 30 s |
| Bupivacain | 1 min 30 s | + | 6 |
| Tramadol | 1 min 20 s | ++ | 4 |

| | | | |
|---|---|---|---|
| ¹Die Klebestärke wurde durch Zug ermittelt. (++): Die Fleischstücke konnten nicht ohne Faserriss voneinander getrennt werden, (+): Bei Zug Riß in der Klebeschicht, ²Suspension ^{[3]}Klebstoff war noch nicht ausgehärtet | | | |

### Beispiel 4: Wirkstoff-Freisetzung

Zur quantitativen Bestimmung der Wirkstofffreisetzung wurde aus 4 g des Prepolymers A, 0,45 g PEG 200, 0,55 g Aspartat B und 250 mg Wirkstoff ein durchsichtiger Film der Dicke 200 µm gerakelt. Daraus wurde ein 5 x 5 cm großes Stück (Gewicht: 0,5 g) ausgeschnitten und in einer Petrischale mit 20 g physiologischer Kochsalzlösung überschichtet und 2 h bei 37°C im Brutschrank gelagert. Die quantitative Bestimmung wurde über HPLC-UV/MS (HPLC-Säule: Inertsil ODS 3 5 µ 120 A 125 mm*2.1 mm 60°C; Eluent A: 25 mmol Ammoniumacetat in Wasser, Eluent B: 25 mmol Ammoniumacetat in Methanol) durchgeführt. Die freigesetzte Wirkstoffmenge Menge ist in Tabelle 2 angegeben.

**Tabelle 2**

| **Wirkstoff** | **Freisetzung in mg/l** | **Freigesetzte Wirkstoffmenge [%]** |
|---|---|---|
| Lidocain | 570 | 45,6 |
| Acetylsalicylsäure | 320 | 25,6 |
| Phenylbutazon | 355 | 28,4 |
| Paracetamol^{][1]} | 136 | 27,2 |
| Cortison^{[1]} | 230 | 46 |
| Naldixinsäure^{[1]} | 110 | 22 |
| Tetracyclin | 547 | 43,8 |
| Chloramphenicol | 646 | 51,7 |

| | | |
|---|---|---|
| [1] eingesetzt wurden in Bsp. 4 100 mg Wirkstoff statt 250 mg | | |

## Patentansprüche

1. Klebstoffsysteme umfassend
A) isocyanatfunktionelle Prepolymere erhältlich aus
A1) aliphatischen Isocyanaten und
A2) Polyolen mit zahlenmittleren Molekulargewichten von ≥ 400 g/mol und mittleren OH-Funktionalitäten von 2 bis 6 B
B) eine Härterkomponente umfassend
B1) aminofunktionelle Asparaginsäureester der allgemeinen Formel (I) 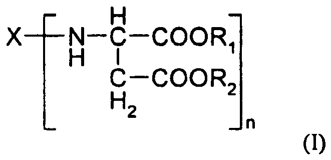 wobei
X ein n-wertiger organischer Rest ist, der durch Entfernung der primären Aminogruppen eines n-wertigen Amins erhalten wird,
R₁, R₂ gleiche oder verschiedene organische Reste sind, die keinen Zerewitinoff-aktiven Wasserstoff aufweisen und
n eine ganze Zahl von mindestens 2 ist
und
B2) organische Füllstoffe, die eine nach DIN 53019 gemessenen Viskosität bei 23°C im Bereich von 10 bis 6000 mPas aufweisen
C) gegebenenfalls aminofunktionelle Umsetzungsprodukte von isocyanatfunktionellen Prepolymeren gemäß der Definition der Komponente A) mit Asparaginsäureestern gemäß Komponente B1) und/oder organischen Füllstoffen gemäß Komponente B2)
und
D) mindestens eine pharmakologisch aktive Verbindung,
**dadurch gekennzeichnet, dass** es sich um einen Gewebekleber für menschliches oder tierisches Gewebe handelt.

2. Klebstoffsysteme gemäß Anspruch 1, **dadurch gekennzeichnet, dass** die in A2) eingesetzten Polyole zahlenmittlere Molekulargewichte von 4000 bis 8500 g/mol aufweisen.

3. Klebstoffsysteme gemäß Anspruch 1 oder 2, **dadurch gekennzeichnet, dass** in A2) Polyalkylenoxid-Polyether eingesetzt werden.

4. Klebstoffsysteme gemäß einem der Ansprüche 1 bis 3, **dadurch gekennzeichnet, dass** als organische Füllstoffe der Komponente B2) Polyetherpolyole eingesetzt werden.

5. Klebstoffsysteme gemäß einem der Ansprüche 1 bis 4, **dadurch gekennzeichnet, dass** als pharmakologisch aktive Wirkstoffe Analgetika mit oder ohne antiinflammatorische Wirkung, Antiphlogistika, antimikrobiell wirksame Substanzen oder Antimykotika eingesetzt werden.

6. Klebstoffsysteme gemäß einem der Ansprüche 1 bis 5, **dadurch gekennzeichnet, dass** anstatt von der Härterkomponente B) ausschließlich zur Härtung der in A) eingesetzten Prepolymere die Umsetzungsprodukte gemäß C) eingesetzt wird.

7. Verfahren zur Herstellung von Klebstoffsystemen gemäß einem der Ansprüche 1 bis 6, bei dem die Komponenten A), B), D) und gegebenenfalls C) in einem Verhältnis von NCO-reaktiven Gruppen zu freien NCO-Gruppen von 1: 1,5 bis 1:1 miteinander vermischt werden.

8. Klebstoffsysteme erhältlich nach Verfahren gemäß Anspruch 7.

9. Verwendung von Klebstoffsystemen gemäß einem der Ansprüche 1 bis 6 oder 8 zur Herstellung eines Mittels zum Verschließen oder Verbinden von Zellgeweben.

10. Klebstofffilme und Verbundteile erhältlich unter Verwendung von Klebstoffsystemen gemäß einem der Ansprüche 1 bis 6 oder 8.

11. 2-Kammerdosiersystem umfassend ein Klebstoffsystem gemäß einem der Ansprüche 1 bis 6 wobei eine Kammer das Prepolymer der Komponente A) und die andere die Härterkomponente B), Wirkstoffkomponente D) und gegebenenfalls C) umfasst.

## Claims

1. Adhesive systems comprising
A) isocyanate group-containing prepolymers obtainable from
A1)aliphatic isocyanates and
A2) polyols with number-averaged molecular weights of ≥ 400 g/mol and average OH group contents of from 2 to 6
B) a curing component comprising
B1)amino group-containing aspartate esters of the general formula (I) 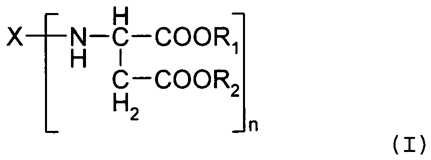 wherein
X is an n-valent organic radical, which is obtained by removal of the primary amino groups of an n-functional amine,
R₁, R₂ are the same or different organic radicals, which contain no Zerevitinov active hydrogen and
n is a whole number of at least 2
and
B2)organic fillers which have a viscosity at 23°C measured to DIN 53019 in the range from 10 to 6000 mPas
C) where appropriate, reaction products of isocyanate group-containing prepolymers according to the definition of component A) with aspartate esters according to component B1) and/or organic fillers according to component B2)
and
D) at least one pharmacologically active compound,
**characterized in that** it is a tissue adhesive for human or animal tissue.

2. Adhesive systems according to Claim 1, **characterized in that** the polyols used in A2) have number-averaged molecular weights of 4000 to 8500 g/mol.

3. Adhesive systems according to Claim 1 or 2, **characterized in that** polyalkylene oxide polyethers are used in A2).

4. Adhesive systems according to one of Claims 1 to 3, **characterized in that** the organic fillers of component B2) used are polyether polyols.

5. Adhesive systems according to one of Claims 1 to 4, **characterized in that** pharmacologically active substances used are analgesics with or without antiinflammatory activity, antiphlogistics, substances with antimicrobial activity or antimycotics.

6. Adhesive systems according to one of Claims 1 to 5, **characterized in that**, instead of the curing component B), the reaction products according to C) is used exclusively for the curing of the prepolymers used in A).

7. Process for the production of adhesive systems according to one of Claims 1 to 6, in which components A), B), D) and optionally C) are mixed with one another in a ratio of NCO-reactive groups to free NCO groups of 1:1.5 to 1:1.

8. Adhesive systems obtainable by the process according to Claim 7.

9. Use of adhesive systems according to one of Claims 1 to 6 or 8 for the production of an agent for the closure or binding of cellular tissues.

10. Adhesive films and laminated parts obtainable with the use of adhesive systems according to one of Claims 1 to 6 or 8.

11. 2-chamber dispensing system containing an adhesive system according to one of Claims 1 to 6, in which one chamber comprises the prepolymer of component A) and the other comprises the curing component B), active ingredient component D) and, where appropriate, C).

## Revendications

1. Systèmes adhésifs, comprenant
A) des prépolymères à fonctionnalité isocyanate, pouvant être obtenus à partir
A1) d'isocyanates aliphatiques et
A2) de polyols présentant des poids moléculaires numériques moyens ≥ 400 g/mole et des fonctionnalités OH moyennes de 2 à 6
B) un composant durcisseur, comprenant
B1) des esters de l'acide aspartique à fonctionnalité amino de formule générale (I) 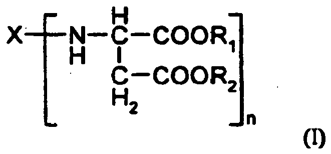 où
X représente un radical organique n-valent, qui est obtenu par élimination des groupes amino primaires d'une amine n-valente,
R₁, R₂ représentent des radicaux organiques identiques ou différents, qui ne présentent pas d'hydrogène actif selon Zerewitinoff et
n représente un nombre entier valant au moins 2,
et
B2) des charges organiques présentant une viscosité, mesurée à 23°C selon la norme DIN 53019, dans la plage de 10 à 6000 mPa.s
C) le cas échéant des produits de transformation à fonctionnalité amino de polymères à fonctionnalité isocyanate selon la définition du composant A) avec des esters de l'acide aspartique selon le composant B1) et/ou des charges organiques selon le composant B2)
et
D) au moins un composé pharmacologiquement actif,
**caractérisés en ce qu'**il s'agit d'une colle de tissus pour des tissus humains ou animaux.

2. Systèmes adhésifs selon la revendication 1, **caractérisés en ce que** les polyols utilisés dans A2) présentent des poids moléculaires numériques moyens de 4000 à 8500 g/mole.

3. Systèmes adhésifs selon la revendication 1 ou 2, **caractérisés en ce qu'**on utilise dans A2) des poly(oxyde d'alkylène)-polyéthers.

4. Systèmes adhésifs selon l'une quelconque des revendications 1 à 3, **caractérisés en ce qu'**on utilise, comme charges organiques du composant B2), des polyétherpolyols.

5. Systèmes adhésifs selon l'une quelconque des revendications 1 à 4, **caractérisés en ce qu'**on utilise comme substances pharmacologiquement actives des analgésiques avec ou sans effet anti-inflammatoire, des antiphlogistiques, des substances à activité antimicrobienne ou des antimycotiques.

6. Systèmes adhésifs selon l'une quelconque des revendications 1 à 5, **caractérisés en ce qu'**on utilise, au lieu du composant durcisseur B), exclusivement, pour le durcissement des prépolymères utilisés dans A), les produits de transformation selon C).

7. Procédé pour la préparation de systèmes adhésifs selon l'une quelconque des revendications 1 à 6, dans lequel les composants A), B), D) et le cas échéant C) sont mélangés dans un rapport de groupes réactifs avec NCO aux groupes NCO libres de 1:1,5 à 1:1.

8. Systèmes adhésifs pouvant être obtenus selon des procédés selon la revendication 7.

9. Utilisation de systèmes adhésifs selon l'une quelconque des revendications 1 à 6 ou 8 pour la préparation d'un agent destiné à fermer ou à joindre des tissus cellulaires.

10. Films adhésifs et pièces composites pouvant être obtenus en utilisant les systèmes adhésifs selon l'une quelconque des revendications 1 à 6 ou 8.

11. Système de dosage à 2 chambres comprenant un système adhésif selon l'une quelconque des revendications 1 à 6, une chambre comprenant le prépolymère du composant A) et l'autre le composant durcisseur B), le composant actif D) et le cas échéant C).
